(19) Europäisches Patentamt / European Patent Office / Office européen des brevets

(11) **EP 3 132 684 B1**

(12) **EUROPEAN PATENT SPECIFICATION**

(45) Date of publication and mention of the grant of the patent: **09.10.2019 Bulletin 2019/41**

(21) Application number: **16188388.9**

(22) Date of filing: **08.05.2013**

(51) Int Cl.:
*A01N 47/34* (2006.01) *A61K 31/17* (2006.01)
*A61P 33/14* (2006.01) *A23K 20/105* (2016.01)
*A23K 50/80* (2016.01) *A61K 9/50* (2006.01)

(54) **TREATMENT OF FISH POPULATIONS WITH LUFENURON**

BEHANDLUNG VON FISCHPOPULATIONEN MITTELS LUFENURON

TRAITEMENT D'UNE POPULATION DE POISSONS AVEC LUFENURON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.05.2012 EP 12167101**

(43) Date of publication of application: **22.02.2017 Bulletin 2017/08**

(62) Document number(s) of the earlier application(s) in accordance with Art. 76 EPC: **13721733.7 / 2 846 640**

(73) Proprietor: **Elanco Tiergesundheit AG**
**4058 Basel (CH)**

(72) Inventors:
- **Blaser, David**
  **Indianapolis, Indiana 46206-6288 (US)**
- **Bouvier, Jacques**
  **Indianapolis, Indiana 46206-6288 (US)**
- **McHenery, John Gerard**
  **Indianapolis, Indiana 46206-6288 (US)**
- **Nanchen, Steve**
  **Indianapolis, Indiana 46206-6288 (US)**
- **Fink, Martin**
  **4056 Basel (CH)**

(74) Representative: **Alexander, Sean Matthew et al**
**Eli Lilly and Company Ltd.**
**Erl Wood Manor**
**Sunninghill Road**
**Windlesham, Surrey GU20 6PH (GB)**

(56) References cited:
**EP-A2- 1 759 692** **WO-A2-99/63824**
**US-A- 5 814 652**

- **GRANT ANDREW N: "Medicines for sea lice", PEST MANAGEMENT SCIENCE, WILEY & SONS, BOGNOR REGIS; GB, vol. 58, June 2002 (2002-06), pages 521-527, XP002592569, ISSN: 1526-498X, DOI: 10.1002/PS.481 [retrieved on 2002-05-07]**
- **EMEA: "Diflubenzuron", , November 1998 (1998-11), pages 1-8, XP002679567, Retrieved from the Internet: URL:http://www.emea.europa.eu/docs/en_GB/document_library/Maximum_Residue_Limits_-_Report/2009/11/WC500013852.pdf [retrieved on 2012-07-10]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 132 684 B1

Printed by Jouve, 75001 PARIS (FR)

## Description

The present invention relates to the control of sea lice, for example *Lepeophtheirus salmonis, Caligus elongatus* and *Caligus rogercresseyi,* infestations in fish farming, which includes the use of the known chitin synthesis inhibitor lufenuron in form of a particular formulation and according to a particular treatment schedule.

The basis of sea louse control in commercial salmonid farming is largely still a treatment with chemicals such as organophosphates, synthetic pyrethroids, chitin synthesis inhibitors, hydrogen peroxide or macrocyclic lactones such as emamectin benzoate. Developing drug resistance by sea lice against commercial products containing compounds from said drug classes presents a big threat to the fish industry; on the one hand higher doses of the compounds might be employed which accelerates the issue of resistance development and moreover has the potential to create environmental toxicology issues. On the other hand there is a desperate search for new chemicals and treatment schedules thereof.

WO99/063824 discloses the use of lufenuron, which is a member of the chemical class of benzoylureas, in the control of sea louse infestations. Lufenuron is proposed exclusively for treatment as an injectable, and protection against sea louse infestation for 128 days after injection is reported. Nevertheless, no commercial lufenuron product has been launched for several reasons. First of all, injecting a huge number of fish at sea is regarded as very cumbersome for the farm personnel and furthermore creates much stress to the fish. In addition, 4 months of effective sea louse control is no longer regarded as sufficient, as the whole cycle of salmon development lasts from 18 months to about 47 months, with an initial phase taking place in fresh water and a consecutive phase, for example lasting from 6 to 23 months, taking place in sea water with exposure to sea lice.

Surprisingly, it has now been found that a very long-lasting and convenient control of sea lice infesting fish, and in particular salmon, may be obtained by an in-feed treatment of the fish with lufenuron before transfer to sea or whilst at sea.

According to the invention, there is provided lufenuron or a veterinary acceptable salt thereof for use in the control of sea lice in a fish population by oral administration, wherein the oral administration comprises administering a daily dose of 1 to 30 mg lufenuron/kg of fish biomass for a time period of 3 to 14 days and wherein the overall amount of lufenuron applied is from 7 to 350 mg/kg of fish biomass during said time period.

Further disclosed is a method for controlling sea lice in a fish population, which comprises feeding lufenuron or a veterinary acceptable salt thereof to said fish population at a daily dose of 1 to 30 mg lufenuron/kg of fish biomass for a time period of 3 to 14 days, and wherein the overall amount of lufenuron applied during said time period is from 7 to 350/ kg of fish biomass.

When applying a treatment schedule according to the present invention, therapeutically effective lufenuron concentrations in the blood, fillet and skin of the fish may be obtained for at least 5 months (150 days), thus indicating an effective protection of the fish against sea lice for a prolonged period of time.

## DETAILED DESCRIPTION

Lufenuron, *N*-{[2,5-Dichlor-4-(1,1,2,3,3,3-hexafluorpropoxy)phenyl]carbamoyl}-2,6-difluorbenzamid, has the chemical formula

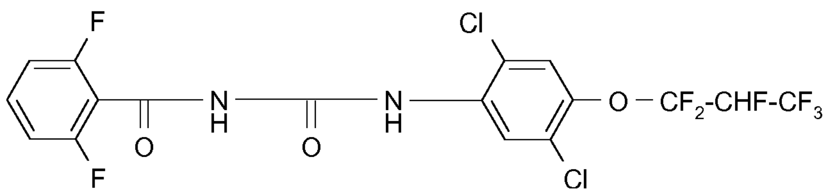

and may be applied in free form or in form of a veterinary acceptable salt. Due to an asymmetrical C-atom being present in the molecule, two enantiomers are in existence. Within the present invention the use of lufenuron in form of the racemic mixture of the two enantiomers is preferred. In addition, racemic lufenuron may exist in various polymorphic forms, for example as polymorph A, B, C or D. Within the present invention, the use of the thermodynamically most stable polymorph A is preferred.

According to one embodiment of the invention the lufenuron is for use in the treatment of salmon, wherein the treatment is performed during the initial fresh water phase before transfer to sea. According to a further embodiment of the invention the lufenuron is for use in the treatment of salmon wherein the treatment is performed whilst the fish are already at sea.

In general, the lufenuron for use in the treatment according to the present invention serves to eliminate, reduce

or prevent, in particular reduce or prevent, sea lice infestation in a fish population. Preferably, the treatment takes places before the fish has been infested with sea lice and have matured. Regarding a lufenuron treatment of salmon, said treatment is performed preferably at the end of the fresh water phase or at the beginning of the seawater phase.

The overall time period for the treatment against sea lice is, for example, from 3 to 14 days, preferably from 5 to 14 days, more preferably from 5 to 10 days and in particular for 7 days (1 week). During the overall treatment time, the feeding of the lufenuron is performed, for example, daily or once every second day, and in particular daily.

The overall amount of lufenuron applied during the treatment is preferably from 14 to 175 mg/kg of fish biomass, more preferably from 20 to 140 mg/kg of fish biomass, even more preferably from 20 to 105 mg/kg fish biomass, and especially from 20 to 84 mg/kg fish biomass. According to a further preferred embodiment of the invention, the overall amount of lufenuron applied during the treatment is from 7 to 175 mg/kg of fish biomass, preferably from 7 to 140 mg/kg of fish biomass, more preferably from 7 to 105 mg/kg fish biomass, and especially from 7 to 70 mg/kg fish biomass.

According to one preferred embodiment of the invention the lufenuron is for daily administration, for a period of time of 3 to 14 days, preferably of 5 to 14 days, more preferably of 5 to 10 days and in particular of 7 days (1 week), wherein the daily dose is from 2 to 25 mg/kg fish biomass, preferably from 3 to 20 mg/kg fish biomass, more preferably from 3 to 15 mg/kg fish biomass. One particularly preferred treatment comprises administering lufenuron for 7 consecutive days with a daily dose of 3 to 6 mg, (total amount of 21 to 42 mg/kg fish biomass) in particular 3 to 5 mg (total amount 21 to 35 mg/kg fish biomass), lufenuron/kg fish biomass. A further particularly preferred treatment comprises administering lufenuron for 7 consecutive days with a daily dose of 6 to 15 mg lufenuron/kg fish biomass (total amount 42 to 105 mg/kg fish biomass) and especially 6 to 12 mg lufenuron/kg fish biomass (total amount 42 to 84 mg (kg fish biomass).

According to another preferred embodiment of the invention the lufenuron is for daily administration, for a period of time of 3 to 14 days, preferably of 5 to 14 days, more preferably of 5 to 10 days and in particular of 7 days (1 week), wherein the daily dose is from 1 to 25 mg/kg fish biomass, preferably from 1 to 20 mg/kg fish biomass, more preferably from 1 to 15 mg/kg fish biomass. One particularly preferred treatment comprises administering lufenuron for 7 consecutive days with a daily dose of 1 to 10 mg, (total amount of 7 to 70 mg/kg fish biomass) in particular 1 to 5 mg (total amount 7 to 35 mg/kg fish biomass), lufenuron/kg fish biomass.

According to a further embodiment of the invention lufenuron is for administration once every second day, for a period of time preferably of 5 to 13 days, more preferably of 5 to 9 days, and in particular of 7 days. A particular treatment comprises treating the fish for a time period of 7 days, administering the feed comprising the lufenuron on days 1, 3, 5 and 7 and withholding any food the day prior to the treatment and on days 2, 4 and 6 of the treatment period. The concentration of lufenuron is adjusted to ensure that the same average dose per kg of fish biomass is administered over the entire treatment period than in a daily treatment. Concerning the overall amount of lufenuron applied during this pulsed treatment, the above given ranges including the preferences apply.

As an example, concerning a 7-day treatment with lufenuron on days 1, 3, 5 and 7, the lufenuron dose on each of said days is, for example, from 2 to 44 mg/kg fish biomass (total amount from 8 to 176 mg/kg fish biomass), advantageously from 3 to 44 mg/kg fish biomass (total amount from 12 to 176 mg/kg fish biomass), preferably from 5 to 35 mg/kg fish biomass (total amount from 20 to 140 mg/kg fish biomass), even more preferably from 5 to 26 mg/kg fish biomass (total amount from 20 to 104 mg/kg fish biomass).

The lufenuron is suitably applied in form of a medicated fish feed. Fish feed is typically present in the form of granules or pellets; common ingredients of said pellets or granules are, for example, fishmeal, fish oil, vegetable proteins, saccharides, such as typical mono- or disaccharides, polysaccharides, such as mannans glucans or alginates, and/or other typical excipients such as pigments, vitamins, minerals, binders and the like. A lufenuron-medicated fish feed may be prepared by incorporating a suitable amount of lufenuron or a salt thereof into the fish feed product. The lufenuron may be incorporated into the feed mixture prior to pelleting. However, it is preferred to coat the pellets or granules with lufenuron. For example, commercially available fish pellets or granules are coated with lufenuron or preferably with a pre-mix containing the lufenuron and one or more veterinary acceptable excipients such as a starch, fumed silica (Aerosil®), microcrystalline cellulose, lactose or the like. In addition, a typical preservative may be present. The concentration of lufenuron in the pre-mix may be chosen within a broad range; for example, a lufenuron concentration of from 0,001 to 90% w/w, preferably from 1 to 50% w/w, and more preferably from 5 to 15% w/w, according to a further embodiment from 0.001 to 10% w/w, preferably from 0.05 to 5% w/w and in particular from 0.15 to 2.5% w/w, based in each case on the entire weight of the pre-mix, has proven as valuable. The feed pellets may be coated with the pre-mix by a dry top-coating method. To this end the pre-mix is added to the pellets, and the resulting mixture is agitated/mixed in order to uniformly distribute the lufenuron onto the pellets. According to an alternative top-coating method with additional oil treatment, to the product of the above-described dry top-coating method is added fish or vegetable oil with continued mixing, until the pellets are thoroughly coated. According to still another embodiment, called vacuum coating method, the pre-mix is first dissolved/suspended in fish or vegetable oil, before being sprayed onto the pellets under vacuum.

Following the addition of the active ingredient to the fish feed, the pellets or granules comprise, for example, from 0.0005 to 5 % (w/w), preferably from 0.001 to 2.5 % (w/w), and in particular from 0.0025 to 1.25 % (w/w) lufenuron, based on the entire weight of the fish feed.

In accordance with this invention lufenuron is excellently suited for use in the control of fish-parasitic crustaceans. These include the Family Caligidae with representative genus *Dissonus, Caligus* (i.e. *C. curtus, C. elongatus*, *C. clemensi*, *C. rogercresseyii*), and *Lepeophtheirus* (i.e. *L. salmonis*); Families Cecropidae, Dichelesthiidae, Lernaeopodidae with representative genus *Salmincola*; Families Pandaridae, Pennellidae with representative genus *Lernaeocera* and *Pennella;* and Family Sphyriidae; Family Lernaeidae with representative genus *Lernaea;* Families Bomolochidae, Chondracanthidae, Ergasilidae and Philichthyidae; Family *Argulidae* with representative genus *Argulus (*i.e. *A. foliaceus*).

The fish include food fish, breeding fish, aquarium, pond, river, reservoir fish of all ages occurring in freshwater, sea water and brackish water. For example, bass, bream, carp, catfish, char, chub, cichlid, cod, eel, flounder, gourami, grayling, grouper, halibut, mullet, plaice, pompano, roach, rudd, salmon, sole, sturgeon, tilapia, trout, tuna, whitefish, yellowtail.

Lufenuron is particularly suitable for treating salmon. The term "salmon" within the scope of this invention will be understood as comprising all representatives of the family Salmonidae, especially of the subfamily salmoninae, and preferably, the Atlantic salmon (*Salmo salar*), rainbow trout (*Oncorhynchus mykiss*)*,* brown or sea trout (*S. trutta*)*,* the Pacific salmon, Cherry salmon or seema (*O. masou*), Taiwanese salmon (*O. masou formosanum*), chinook salmon or King salmon (*O. tshawytscha*)*,* chum salmon or Calico salmon (*O. keta*), coho salmon or silver salmon (*O. kisutch*), pink salmon (*O. gorbuscha*), Sockeye salmon or Red salmon (*O. nerka*)*,* artificially propagated species, such as *Salmo clarkii,* and *Salvelinus* species such as Brook trout (*S. fontinalis*)*.*

Preferred objects of the present invention are the Atlantic and Pacific salmon and the sea trout including trout species, which are farmed at sea but not traditionally called "sea trout".

When lufenuron is applied according to a schedule according to the present invention, the fish will absorb the lufenuron such that a therapeutically effective concentration of the active substance will be maintained for a prolonged time, for example for at least 5 months, preferably for at least 6 months and more preferably for at least 9 months. Trials have shown that the protection period of the fish against sea lice corresponds very well with the observed lufenuron levels in the fish fillet or blood.

While the treatment with lufenuron alone according to the treatment schedule of the present invention provides in general a complete protection against sea lice for extended periods of time, said treatment may in certain circumstances be further improved by the use of lufenuron in combination with either another sea louse controlling agent; or a vaccine component including immune enhancing agents; or a feed ingredient containing immune modifying agents. Such combination treatments might be required where the fish have already been infested with parasites, which have matured before the lufenuron treatment, or in case rapid clearance of the parasites is desired.

Suitable further sea louse controlling agents are, for example, hydrogen peroxide; formaldehyde; an organophosphate such as trichlorfon, malathion, dichlorvos or azamethiphos; a macrocyclic lactone such as ivermectin, emamectin benzoate or moxidectin; a pyrethroid such as cypermethrin, in particular cypermethrin *cis*-40 : *trans-60* or high *cis* cypermethrin *cis*-80 : *trans*-20, or deltamethrin; a neonicotinoid such as imidacloprid, nitenpyram, thiamethoxam or thiacloprid; a spinosyn such as spinosad; an insect juvenile hormone analogue such as epofenonane, triprene, methoprene, hydroprene or kinoprene; or a carbamate such as phenoxycarb.

If lufenuron is used in combination with another compound being active in the control of sea lice, said combination partner is preferably an organophosphate, a pyrethroid such as cypermethrin or deltamethrin, a macrocyclic lactone such as emamectin benzoate; hydrogen peroxide; or a neonicotinoid such as thiacloprid.

In addition, it is preferred to apply lufenuron according to the schedule according to the present invention in the absence of a further sea louse controlling agent selected from the group consisting of a compound of formula

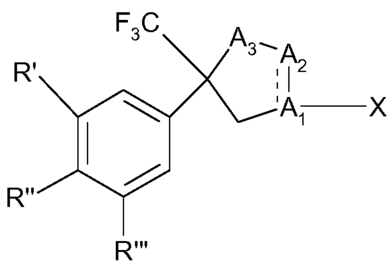

(I)

including all geometric and stereoisomers, N-oxides, S-oxides and salts thereof according to WO2011/157733, wherein $A_1$, $A_2$, $A_3$, R', R", R''' and X each have the meaning as given on pages 1 and 2 of said WO2011/157733; and, in particular, wherein

R', R" and R''' are each independently hydrogen, halogen, cyano, $C_1$-$C_2$-alkyl, halo-$C_1$-$C_2$-alkyl, $C_1$-$C_2$-alkoxy or $C_1$-$C_2$-haloalkoxy, subject to the proviso that at least one of R', R" and R''' is not hydrogen;

$A_1$ is C, $A_2$ is N, $A_3$ is O, $CH_2$ or $NR_1$' and the bond between $A_1$ and $A_2$ is a double bond; or $A_1$ is N, $A_2$ and $A_3$ are each $CH_2$ and the bond between $A_1$ and $A_2$ is a single bond;
$R_1$' independently is as defined as $R_1$ below; and X is

(a) a radical of formula

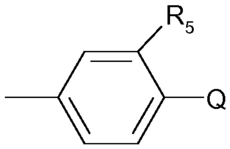

(II),

wherein $R_5$ is H, $C_1$-$C_2$-alkyl, $C_1$-$C_2$-haloalkyl, halogen, nitro or cyano and Q is

(i) a 5- or 6-membered heteroaromatic ring comprising 1 to 3 same or different heteroatoms selected from the group consisting of O, S and N; or is
(ii) a group -C(O)N($R_1$)-T, wherein $R_1$ is H, $C_1$-$C_4$-alkyl, $C_2$-$C_4$-alkylcarbonyl or $C_2$-$C_4$-alkoxycarbonyl and T is $C_1$-$C_6$-alkyl which is unsubstituted or substituted by $C_3$-$C_6$-cycloalkyl, halogen, cyano, nitro, amino, hydroxy, $C_1$-$C_6$-alkoxy, $C_1$-$C_6$-haloalkoxy, $C_1$-$C_6$-alkylthio, $C_1$-$C_6$-haloalkylthio, $C_1$-$C_6$-alkylsulfinyl, $C_1$-$C_6$-haloalkylsulfinyl, $C_1$-$C_6$-alkylsulfonyl, $C_1$-$C_6$-haloalkylsulfonyl, carboxy, carbamoyl, $C_1$-$C_6$-alkylcarbonylamino, $C_1$-$C_6$-haloalkyl-carbonylamino, $C_1$-$C_6$-alkoxycarbonyl, sulfonamido, N-mono- or N,N, di-$C_1$-$C_4$-alkylsulfonamido, $C_2$-$C_6$-alkanoyl, unsubstituted or in the alkyl portion by halogen, cyano, ethenyl or ethynyl substituted N-$C_1$-$C_6$-alkylaminocarbonyl, or unsubstituted or halogen-, $C_1$-$C_2$-alkyl-, $C_1$-$C_2$-haloalkyl or cyano-substituted 4- to 6-membered heterocyclyl; or T is $C_3$-$C_6$-cycloalkyl or 4- to 6-membered heterocyclyl, which is each unsubstituted or substituted by halogen, $C_1$-$C_2$-alkyl, $C_1$-$C_2$-haloalkyl or cyano; or is
(iii) a radical -C(O)NH-C=N-O-$C_1$-$C_2$-alkyl, a radical -C(O)N=C-N-di-$C_1$-$C_2$-alkyl or a radical -C(O)N=C($NH_2$)-O-$C_1$-$C_2$alkyl; or is
(iv) a group -CH($R_3$)-N($R_4$)-C(O)-$T_1$, wherein $R_3$ is H, $C_1$-$C_6$-alkyl, $C_1$-$C_6$-haloalkyl, halogen or cyano, $R_4$ is H; $C_1$-$C_4$-alkyl, $C_2$-$C_4$-alkylcarbonyl or $C_2$-$C_4$-alkoxycarbonyl, and $T_1$ is independently defined as T above;

(b) a radical of formula

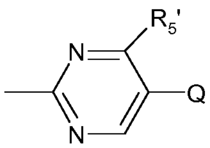

(III),

wherein $R_5$' is H, $C_1$-$C_2$-alkyl, $C_1$-$C_2$-haloalkyl, halogen, nitro or cyano, and Q is as defined above;
(c) a radical of formula

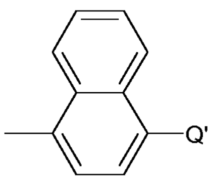

(IV),

wherein Q' is a radical as defined in embodiments (ii), (iii) and (iv) for Q above; or
(d) a radical of formula

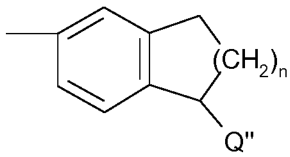

(V),

wherein n is 1 or 2 and Q" is a group $-N(R_4)-C(O)-T_2$, wherein $T_2$ independently has the meaning of T above and $R_4$ is as defined above.

A suitable combination treatment with lufenuron and another sea louse-controlling agent may be performed, for example, by treating the fish, in particular salmon, initially with lufenuron according to the in-feed method and regime as mentioned above, and thereafter, for example 3 months, preferably 5 months, more preferably 6 months and in particular 9 months following the end of the lufenuron in-feed treatment performing a treatment with the additional sea louse controlling agent; said second treatment may be a bath treatment, an in-feed treatment or preferably a treatment by injecting the additional sea louse controlling agent to the fish. According to a preferred embodiment of this combination treatment, the in-feed treatment with the lufenuron takes place at the end of the fresh water phase of salmon evolution or at the beginning of their sea water phase.

A further combination treatment comprises first of all treating the fish, in particular salmon, with the additional sea louse controlling agent and thereafter, for example 1 hour to 2 months thereafter, preferably 1 hour to 1 month thereafter or in particular 1 week to 1 month thereafter, performing a lufenuron in-feed treatment according to the present disclosure as described above. According to a preferred embodiment of this combination treatment, the treatment with the additional sea louse controlling agent is a bath treatment, an in-feed treatment or injectable treatment which takes place at the beginning of the sea water phase, for example 1 hour to 3 months, preferably 6 hours to 2 months and in particular 12 hours to 1 month following the release of the fish to sea water.

According to a further embodiment of the disclosure, the in-feed treatment with lufenuron is combined with a vaccination of the fish against typical bacterial or viral infections. Typical bacterial diseases to be treated by vaccination are, for example, vibrosis, furunculosis, wound diseases, atypical aeromonas salmonicida, piscirickettsiosis or ERM/yersiniosis. Examples of viral diseases to be treated are pancreas disease/PDV, infectious pancreatic necrosis/IPNV or infectious salmon anemia/ISAV. The vaccine is in general applied by a bath or in-feed treatment or preferably by injection. The vaccination may take place either shortly before, during or after the lufenuron in-feed treatment of the fish.

According to still a further embodiment of the invention, the in-feed treatment with lufenuron is combined with a feed ingredient, which has modulatory effect on the biology, physiology, biochemistry and, particularly, immunology of the fish. Typical functions might be increased secretion of mucus, or changes in the characteristics of the mucus, such that the exposure of the parasites to the lufenuron is enhanced, or the lice exposed to the lufenuron are less able to adhere to the treated fish. The use of the modulatory in-feed ingredient may take place over extended periods in the fish production cycle or may take place before, during or after the treatment with lufenuron. Examples of such modulatory ingredients are glucans, mannans or alginates either used alone or in combination with vitamins and/or minerals.

The following Examples further illustrate the invention.

Example 1:

Three groups of 150 salmon (Atlantic Salmon, *S. salar*) of average weight 238 g were treated with medicated pellets containing lufenuron at a dose of respectively 3, 5 and 10 mg/kg/day during 7 consecutive days. The medicated pellets were prepared by dry coating commercially available fish feed pellets with a lufenuron-containing pre-mix to reach a content of 0.03, 0.05 and 0.1% (w/w) lufenuron in the fish feed and administered at a 1% feeding rate. The treatment was carried out in sea water.

Each group was maintained in a separate cage. A fourth cage containing 150 salmon of average weight 238 g was added to the study as a non-treated control group. After treatment, the fish were exposed to natural sea louse infestation for a period of 9 months. During the study the water temperature varied between 4 and 12 °C. Fish weight and sea louse numbers were assessed 1 day post-treatment, 1 week post-treatment and afterwards on a monthly basis until month 9. Counts were conducted on 10 fish at each sampling occasion. The numbers of chalimus, pre-adults and adult male, mobile and mature female *L. salmonis* were recorded for each fish. No distinction was made between chalimus stages I-IV or between pre-adult and adult male stages.

Efficacy was calculated using the formula:

% Efficacy = 100 - (100 x mean of treatment group / mean of control).

As shown in Table 1, the 3 mg/kg treatment protected the fish against sea louse infestation until 5 months post-treatment. Chalimus and pre-adult stages were controlled, and this in turn prevented an accumulation of adult stages. At month 6, efficacy against pre-adult lice fell below that of the 5 mg/kg/day and 10 mg/kg/day dose.

Table 1: 3 mg/kg group *L. salmonis* average group counts

| 3 mg/kg group | | *L. salmonis*: all stages | | *L. salmonis:* pre-& adults | | |
|---|---|---|---|---|---|---|
| Time | Weight [g] | Treated | *Control* | Treated | *Control* | Efficacy % |
| 1 day | 263 | 0.10 | *0.10* | 0.10 | *0.00* | n/a |
| 1 week | 283 | 0.10 | *0.40* | 0.10 | *0.00* | n/a |
| 1 month | 409 | 0.00 | *1.00* | 0.00 | *1.00* | 100 |
| 2 month | 600 | 0.30 | *2.90* | 0.10 | *1.90* | 95 |
| 3 month | 915 | 0.20 | *3.10* | 0.10 | *2.20* | 95 |
| 4 month | n/a | 0.00 | *2.50* | 0.00 | *2.40* | 100 |
| 5 month | 1410 | 0.10 | *3.60* | 0.10 | *3.60* | 97 |
| 6 month | 1797 | 1.30 | *2.60* | 1.30 | *2.60* | 50 |

As shown in Table 2, the 5 mg/kg treatment protected the fish against sea louse infestation until 9 months post treatment. Chalimus and preadult and adult stages were controlled.

Table 2: 5 mg/kg group *L. salmonis* average group counts

| 5 mg/kg group | | *L. salmonis:* all stages | | *L. salmonis:* pre-& adults | | |
|---|---|---|---|---|---|---|
| Time | Weight [g] | Treated | *Control* | Treated | *Control* | Efficacy |
| 1 day | 253 | 0.00 | *0.10* | 0.00 | *0.00* | n/a |
| 1 week | 294 | 0.00 | *0.40* | 0.00 | *0.00* | n/a |
| 1 month | 407 | 1.90 | *1.00* | 0.10 | *1.00* | 90 |
| 2 month | 701 | 0.00 | *2.90* | 0.00 | *1.90* | 100 |
| 3 month | 1000 | 0.20 | *3.10* | 0.20 | *2.20* | 91 |
| 4 month | 1241 | 0.10 | *2.50* | 0.10 | *2.40* | 96 |
| 5 month | 1593 | 0.10 | *3.60* | 0.10 | *3.60* | 97 |
| 6 month | 1655 | 0.60 | *2.60* | 0.60 | *2.60* | 77 |
| 7 month | 1995 | 1.80 | *5.30* | 0.60 | *5.10* | 88 |
| 8 month | 1980 | 0.50 | *5.40* | 0.50 | *5.10* | 90 |
| 9 month | 2290 | 0.40 | *6.80* | 0.40 | *6.80* | 94 |

As shown in Table 3, the 10 mg/kg treatment protected the fish against sea louse infestation up to the end of the study which was 9 months post treatment. Chalimus, preadult and adult stages were controlled. Efficacy of the 10 mg/kg group was superior to the 5mg/kg group since at all time points the efficacy against sea lice was more than 95%.

Table 3: 10 mg/kg group *L. salmonis* average group counts

| 10 mg/kg group | | *L. salmonis:* all stages | | *L. salmonis:* pre-& adults | | |
|---|---|---|---|---|---|---|
| Time | Weight [g] | Treated | *Control* | Treated | *Control* | Efficacy |
| 1 day | 256 | 0.00 | *0.10* | 0.00 | *0.00* | n/a |

(continued)

| 10 mg/kg group | | *L. salmonis:* all stages | | *L. salmonis:* pre-& adults | | |
|---|---|---|---|---|---|---|
| Time | Weight [g] | Treated | *Control* | Treated | *Control* | Efficacy |
| 1 week | 296 | 0.20 | *0.40* | 0.00 | *0.00* | n/a |
| 1 month | 423 | 3.10 | *1.00* | 0.00 | *1.00* | 100 |
| 2 month | 644 | 0.00 | *2.90* | 0.00 | *1.90* | 100 |
| 3 month | 974 | 0.10 | *3.10* | 0.10 | *2.20* | 95 |
| 4 month | 1161 | 0.00 | *2.50* | 0.00 | *2.40* | 100 |
| 5 month | 1440 | 0.00 | *3.60* | 0.00 | *3.60* | 100 |
| 6 month | 1628 | 0.30 | *2.60* | 0.10 | *2.60* | 96 |
| 7 month | 1905 | 0.80 | *5.30* | 0.10 | *5.10* | 98 |
| 8 month | 2091 | 0.10 | *5.40* | 0.10 | *5.10* | 98 |
| 9 month | 2065 | 0.00 | *6.80* | 0.00 | *6.80* | 100 |

Example 2:

Three groups of 400 Atlantic salmon (*Salmo salar*) of average weight 80g were treated with medicated pellets containing lufenuron to deliver doses equivalent to 1, 5 and 10 mg/kg/day over 7 consecutive days. A fourth group containing 400 salmon of average weight 80g was added to the study as a non-treated control group. The medicated pellets were prepared by coating commercially available fish feed pellets with a lufenuron-containing pre-mix to reach a content of 0.005, 0.025 and 0.05% (w/w) lufenuron in the fish feed. The groups were randomized and the identity of the feeds blinded to the site staff. The treatment was undertaken in a fresh water hatchery and the fish transferred to sea water within 7 days of completion of treatment.

On transfer to sea each group was split into two cages and all cage groups were maintained in separate randomized cages.

After treatment, the fish were exposed to natural sea louse infestation. During the study the temperature varied between 1.9 and 8.1 °C. Fish weights were assessed at allocation to cages, again approximately 2 weeks after the sea transfer and then at 4 weekly intervals. Sea louse numbers were assessed at approximately 2 weeks after the sea transfer and then at 4 weekly intervals. The numbers of chalimus, pre-adults and adult male, mobile and mature female *L. salmonis* were recorded for each fish. No distinction was made between chalimus stages I-IV or between pre-adult and adult male stages. Total numbers of *Caligus elongatus* were also recorded but no significant infestation was established to enable an assessment of efficacy.

Efficacy was calculated using the formula:

$$\% \text{ Efficacy} = 100 - (100 \times \text{mean of treatment group} / \text{mean of control}).$$

Table 4: 1 mg/kg group *L. salmonis* counts

| 1 mg/kg group | | *L. salmonis:* all stages | | *L. salmonis:* pre-& adults | | |
|---|---|---|---|---|---|---|
| Days post sea transfer | Weight [g] | Treated | *Control* | Treated | *Control* | Efficacy % |
| 5 | 109 | | | | | |
| 15 | 98 | 0 | *0.4* | 0 | *0.25* | 100 |
| 42 | 135 | 0 | *1.25* | 0 | *0.9* | 100 |
| 76 | 196 | 0.1 | *2.7* | 0.1 | *2.65* | 96.2 |
| 98 | 258 | 0.8 | *4.15* | 0.45 | *2.8* | 83.9 |
| 125 | 303 | 0.4 | *4.9* | 0.3 | *4.25* | 92.9 |

(continued)

| 1 mg/kg group | | *L. salmonis:* all stages | | *L. salmonis:* pre-& adults | | |
|---|---|---|---|---|---|---|
| Days post sea transfer | Weight [g] | Treated | *Control* | Treated | *Control* | Efficacy % |
| 154 | 358 | 0.3 | *4.2* | 0.3 | *4.15* | 92.8 |

As shown in Table 4, it can be concluded that the 1 mg/kg treatment protected the fish against sea louse infestation until at least the five month post-treatment with approximately 90% efficacy. Chalimus, pre-adult and adult stages were controlled.

Table 5: 5 mg/kg group *L. salmonis* counts

| 5 mg/kg group | | *L. salmonis:* all stages | | *L. Salmonis:* pre-& adults | | |
|---|---|---|---|---|---|---|
| Days post sea transfer | Weight [g] | Treated | *Control* | Treated | *Control* | Efficacy % |
| 5 | 106 | | | | | |
| 15 | 107 | 0.1 | *0.4* | 0 | *0.25* | 100 |
| 76 | 201 | 0 | *2.7* | 0 | *2.65* | 100 |
| 98 | 277 | 0.1 | *4.15* | 0.1 | *2.8* | 96.4 |
| 125 | 318 | 0 | *4.9* | 0 | *4.25* | 100 |
| 154 | 371 | 0 | *4.2* | 0 | *4.15* | 100 |

As shown in Table 5, it can be concluded that the 5 mg/kg treatment protected the fish against sea louse infestation until 5 months post-treatment. The study continues. It is possible that the protection will be longer. Chalimus, pre-adult and adult stages were controlled.

Table 6: 10 mg/kg group *L. salmonis* counts

| 10 mg/kg group | | *L. salmonis:* all stages | | *L. salmonis:* pre-& adults | | |
|---|---|---|---|---|---|---|
| Days post sea transfer | Weight [g] | Treated | *Control* | Treated | *Control* | Efficacy % |
| 5 | 110 | | | | | |
| 15 | 115 | 0.5 | *0.4* | 0 | *0.25* | 100 |
| 76 | 208 | 0 | *2.7* | 0 | *2.65* | 100 |
| 98 | 291 | 0 | *4.15* | 0 | *2.8* | 100 |
| 125 | 316 | 0 | *4.9* | 0 | *4.25* | 100 |
| 154 | 384 | 0 | *4.2* | 0 | *4.15* | 100 |

As shown in Table 6, it can be concluded that the 10 mg/kg treatment protected the fish against sea louse infestation up to 5 months post-treatment. The study continues. It is possible that the protection will be longer. Chalimus, pre-adult and adult stages were controlled.

## Claims

1. Lufenuron or a veterinary acceptable salt thereof for use in the control of sea lice in a fish population by oral administration, wherein the oral administration comprises administering a daily dose of 1 to 30 mg lufenuron/kg of fish biomass for a time period of 3 to 14 days and wherein the overall amount of lufenuron applied is from 7 to 350 mg/kg of fish biomass during said time period.

2. Lufenuron or a veterinary salt thereof for use according to claim 1, wherein the oral administration comprises administering a medicated fish feed comprising the lufenuron to the fish population.

3. Lufenuron or a veterinary salt thereof for use according to claim 2, wherein the medicated fish feed is in the form of fish feed granules or pellets which are coated with lufenuron.

4. Lufenuron or a veterinary salt thereof for use according to any one of claims 1 to 3, wherein the lufenuron is administered to the fish population daily for 5 to 10 days.

5. Lufenuron or a veterinary salt thereof for use according to any one of claims 1 to 4, wherein lufenuron is administered to the fish population at a daily dose of from 1 to 20 mg/kg fish biomass.

6. Lurenuron or a veterinary salt thereof for use according to claim 5, wherein lufenuron is administered to the fish population at a daily dose of from 1 to 15 mg/kg fish biomass.

7. Lufenuron or a veterinary salt thereof for use according to any one of claims 1 to 6, wherein the fish population comprises salmons.

## Patentansprüche

1. Lufenuron oder ein tiermedizinisch zulässiges Salz davon zur Verwendung bei der Bekämpfung von Seeflöhen in einer Fischpopulation mittels oraler Verabreichung, wobei die orale Verabreichung das Verabreichen einer Tagesdosis von 1 bis 30 mg Lufenuron pro Kilogramm Fisch-Biomasse über einen Zeitraum von 3 bis 14 Tagen umfasst und wobei die Gesamtmenge an verwendeten Lufenuron zwischen 7 bis 350 mg pro Kilogramm Fisch-Biomasse während dieses Zeitraums beträgt.

2. Lufenuron oder ein tiermedizinisch zulässiges Salz davon zur Verwendung gemäß Anspruch 1, wobei die orale Verabreichung das Verabreichen eines arzneihaltigen Fischfutters, das Lufenuron aufweist, an die Fischpopulation umfasst.

3. Lufenuron oder ein tiermedizinisch zulässiges Salz davon zur Verwendung gemäß Anspruch 2, wobei das arzneihaltige Fischfutter in Form von Fischfuttergranulaten oder -pellets vorliegt, die mit Lufenuron ummantelt sind.

4. Lufenuron oder ein tiermedizinisch zulässiges Salz davon zur Verwendung gemäß einem der Ansprüche 1 bis 3, wobei das Lufenuron an die Fischpopulation täglich über 5 bis 10 Tage verabreicht wird.

5. Lufenuron oder ein tiermedizinisch zulässiges Salz davon zur Verwendung gemäß einem der Ansprüche 1 bis 4, wobei Lufenuron an die Fischpopulation in einer Tagesdosis zwischen 1 bis 20 mg pro Kilogramm Fisch-Biomasse verabreicht wird.

6. Lufenuron oder ein tiermedizinisch zulässiges Salz davon zur Verwendung gemäß Anspruch 5, wobei Lufenuron an die Fischpopulation in einer Tagesdosis zwischen 1 bis 15 mg pro Kilogramm Fisch-Biomasse verabreicht wird.

7. Lufenuron oder ein tiermedizinisch zulässiges Salz davon zur Verwendung gemäß einem der Ansprüche 1 bis 6, wobei die Fischpopulation Lachs umfasst.

## Revendications

1. Lufénuron ou un de ses sels acceptables du point de vue vétérinaire pour son utilisation dans la lutte contre le pou du poisson dans une population piscicole via une administration par voie orale, dans lequel l’administration par voie orale comprend l’administration d’une dose quotidienne de 1 à 30 mg de lufénuron/kg de biomasse de poisson pendant un laps de temps de 3 à 14 jours, et dans lequel la quantité globale de lufénuron appliqué s’élève de 7 à 350 mg/kg de biomasse de poisson au cours dudit laps de temps.

2. Lufénuron ou un de ses sels acceptables du point de vue vétérinaire pour son utilisation selon la revendication 1, dans lequel l’administration par voie orale comprend l’administration d’un aliment médicamenteux pour poisson comprenant le lufénuron à la population piscicole.

3. Lufénuron ou un de ses sels acceptables du point de vue vétérinaire pour son utilisation selon la revendication 2,

dans lequel l'aliment médicamenteux pour poisson se présente sous la forme de granulés ou de pastilles d'aliment pour poisson, qui sont enrobés de lufénuron.

4. Lufénuron ou un de ses sels acceptables du point de vue vétérinaire pour son utilisation selon l'une quelconque des revendications 1 à 3, dans lequel le lufénuron est administré à la population piscicole quotidiennement pendant 5 à 10 jours.

5. Lufénuron ou un de ses sels acceptables du point de vue vétérinaire pour son utilisation selon l'une quelconque des revendications 1 à 4, dans lequel le lufénuron est administré à la population piscicole à raison d'une dose quotidienne de 1 à 20 mg/kg de biomasse de poisson.

6. Lufénuron ou un de ses sels acceptables du point de vue vétérinaire pour son utilisation selon la revendication 5, dans lequel le lufénuron est administré à la population piscicole à raison d'une dose quotidienne de 1 à 15 mg/kg de biomasse de poisson.

7. Lufénuron ou un de ses sels acceptables du point de vue vétérinaire pour son utilisation selon l'une quelconque des revendications 1 à 6, dans lequel la population piscicole comprend des saumons.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 99063824 A
- WO 2011157733 A